# EUROPEAN PATENT SPECIFICATION

(11) **EP 0 637 958 B1**
(45) Date of publication and mention of the grant of the patent: **02.04.1997**
(21) Application number: 93909847.1
(22) Date of filing: 27.04.1993
(51) Int. Cl.: A61K 31/225, A61K 31/195, C07C 233/47, C07C 237/22

(54) **ANTI-OBESITY DRUGS**
WIRKSTOFFE GEGEN FETTLEIBIGKEIT
MEDICAMENTS CONTRE L'OBESITE

(30) Priority: 28.04.1992 IL 10170892
(43) Date of publication of application: 15.02.1995
(73) Proprietor: SENYORINA LTD., 45 421 Herzlia (IL)
(72) Inventor: SHINITZKY, Meir, 46 910 Kfar Shmaryahu (IL); Schenfeld, Avner, Rehovot 76346 (IL)
(74) Representative: VOSSIUS & PARTNER
(86) International application number: EP9301014
(87) International publication number: WO9321913

(56) References cited:
- DE-A- 2 234 399
- US-A- 4 711 896
- J. CHEM. SOC. CHEM. COMMUN., no. 9, 1986, pages 659-661; R.T.C. BROWNLEE: 'The synthesis and characterization of a series of bis-intercalating bis- anthracyclines'
- KHIM.-FARM. ZH., vol. 26, no. 2, 1992, pages 43-45; N.A. GRIGORYAN: 'Synthesis and antistaphylococcal activity of dicarboxylic acid derivatives containing an amino acid fragment', p. 44, abstract
- DIABETES, vol. 37, 1988, pages 1618-1624; R. TZUR ET AL.: 'Hypolipidemic, antiobesity, and hypoglycemic-hypoinsulinemic effects of beta,beta'-methyl- substituted hexadecanedioic acid in sand rats'

## Description

The present invention is in the field of treatment and prevention of obesity. The present invention provides compositions containing as active ingredient, lypophilic derivatives of natural amino acids and their use in the treatment or prevention of such disorders.

The following prior art is believed to be relevant as a background to the present invention:
**1.** Bar-Tana *et al., J. Biol. Chem.,* 1985, **260**, 8404-8410.
**2.** Rose-Kahn *et al., J. Biol. Chem.,* 1985, **260**, 8411-8415.
**3.** Bar-Tana *et al., J. Lipid Res.,* 1988, **29,** 431-441.
**4.** Frenkel *et al., J. Biol. Chem.,* 1988, **263**, 8491-8497.
**5.** Tzur *et al., Diabetes,* 1988, **37**, 1618-1624.
**6.** U.S. Patent No. 4,634,795.
**7.** U.S. Patent No. 4,689,344.
**8.** U.S. Patent No. 4,711,896.
**9.** U.S. Patent No. 4,908,385.

In the following text, reference to these prior art publications will be made by indicating in brackets their number from the above list.

Energy from food is primarily provided by carbohydrates and lipids. Carbohydrates usually supply the immediate energy needs and their excess is stored as glycogen in the liver or converted to lipids. Lipids can also be metabolized as immediate energy providing substances but their rate of energy provision is relatively slow and they are generally stored in the body for use in states of deprivation. Lipid is stored in the body mostly as fat under the skin and consumption of lipids and carbohydrates beyond the metabolic need leads to fattening. The associated medical and aesthetic problems, are a major concern in modern society.

Apart from surgery and dietary means for reduction of fat absorption in the small intestine, there are presently no satisfactory means for reducing fat storage in the body and the current means of choice are still diet and exercise. There is, however, a desire for drugs which will reduce fat accumulation by inhibiting lipid and lipoprotein synthesis in the liver. Recently, a series of β,β' tetramethyl substituted α,ω dicarboxylic acids (MEDICA) have been synthesized and suggested as potential anti-fattening drugs^{(1-4,6-9)}. The most potent drug of this series was found to be the hexadecane derivative (MEDICA 16). It was demonstrated that MEDICA, which is a non-naturally occurring fatty acid, could inhibit biosynthetic pathways of triglycerides and cholesterol in the liver. Experiments with MEDICA 16 given in the diet to normal and obese rats have indicated a strong inhibition of glyceride and cholesterol biosynthesis in the liver evidenced by a marked reduction in their serum contents⁽³⁾. Furthermore, in the obese animals adipose tissue was reduced by about 75% over the whole body concomitantly to extensive weight loss⁽⁵⁾. However, the metabolic clearance of these compounds via integration into glycerol esters or via oxidation is relatively slow due to the presence of carboxylate at the two edges of the molecules and the β alkyl substitution. Despite their impressive effect, MEDICA are expected to exert a long term toxicity due to their non-compatible molecular structure. Thus, chronic intake of MEDICA, which is required for maintaining a low fat state, would likely be associated with adverse toxic effects in the long run.

It is the object of the present invention to provide a pharmaceutical composition, method and dietary supplements for the treatment and/or prevention of obesity. More specifically, it is an object of the present invention to provide such composition and method utilizing lipophilic derivatives of natural amino acids.

It is a further object of the present invention to provide certain novel lipophilic derivatives of natural amino acids useful in such compositions and methods.

The remaining objects of the present invention will be illustrated from the following description and claims.

In one of its aspects, the present invention provides a pharmaceutical composition for the treatment of obesity comprising a pharmaceutically acceptable carrier and, as an active ingredient, a compound having the general Formula I:

R₄-(CH₂)ₙ-CO-N(R₁)-CH(R₂)-CO(R₃) (I)

- wherein: R₁ represents H or CH₃;
R₂ represents a side chain of a naturally occuring amino acid;
R₃ represents OH, OCH₂CH₃ and NH₂;
n is 6 - 18, preferably 12 - 16; and
R₄ represents a group having the general Formula II:

R₃-CO-CH(R₂)-N(R₁)-CO- (II)
- wherein: R₁, R₂ and R₃ have the above meanings.

The present invention also provides the use of an active ingredient for the preparation of a pharmaceutical composition for the treatment of obesity, or of a dietary supplement to be administered to obese individuals, said active ingredient being a compound having the following general Formula I:

R₄-(CH₂)ₙ-CO-N(R₁)-CH(R₂)-CO(-R₃) (I)

- where: R₁ represents H or CH₃;
R₂ represents a side chain of a naturally occurring amino acid;
R₃ represents OH, OCH₂CH₃ and NH₂;
n is 6 - 18; and
R₄ represents CH₃ or a group having the general Formula II:

R₃-CO-CH(R₂)-N(R₁)-CO- (II)
- where: R₁, R₂ and R₃ have the above meanings.

An example of compounds of Formula I wherein R₄ is CH₃, is N-palmitoyl sarcosine (P-Sar) having the Formula III:

CH₃-(CH₂)₁₄-CO-N(CH₃)-CH₂COOH (III)

Examples of compounds of Formula I, in which R₄ represents a group having the general Formula II, are N,N' sebacoyl bis-glycine (GSG), having the Formul IV:

HOOC-CH₂-NH-CO-(CH₂)₈-CO-NH-CH₂-COOH (IV)

N,N' sebacoyl bis ℓ-aspargin (NSN), having the Formula V:

(CH₂)₈-[CO-NH-CH(CH₂CONH₂)-COOH]₂ (V)

N,N' sebacoyl bis-sarcosine [S(Sar)₂] having the Formula VI:

(CH₂)₈-[CO-N(CH₃)-CH₂-COOH]₂ (VI)

N,N' sebacoyl bis-sarcosine-ethylester [S(SarOEt)₂], having the Formula VII:

(CH₂)₈-[CO-N(CH₃)-CH₂-COO-C₂H₅]₂ (VII)

and
N,N' sebacoyl bis-phenylalanine (FSF), having the Formula VIII:

(CH₂)₈-[CO-NH-CH(CH₂-C₅h₆)-COOH]₂. (VIII)

### BRIEF DESCRIPTION OF THE DRAWINGS

**Fig. 1** shows the effect of NSN (0.1% w/w in purina) on weight of 3 months old mice during a 1 month *ad lib.* feeding;
**Fig. 2** shows the effect of several anti-fattening agents including a fattening diet in accordance with the invention (0.1% w/w in purina + 6% corn oil) on weight of 3 months old mice;
**Fig. 3** shows the daily weight of adult mice administered with various anti-fattening agents of the invention; and
**Fig. 4** shows the effect of treatment with NSN on the ³H₂O incorporation to liver and adipose tissue of adult mice.

The present invention will now be described with reference to the following non-limiting examples.

### Example 1: preparation of compounds

Lypophilic derivatives of the natural amino acids can be synthesized via the N-hydroxy succinimide ester of the respective fatty acid. Such active esters react with primary or secondary amine to form the corresponding amide while liberating a free N-hydroxy succinimide.

In the following are three examples of such synthesis:

### A: preparation of P-Sar

Hydroxy succinimide ester of palmitic acid (PHS) was obtained from SIGMA. One volume of 30mM PHS in tetrahydrofuran was mixed with one volume of excess sarcosine (0.3 M) in 0.1 M of aqueous sodium bicarbonate according to the procedure described by Lapidot *et al., J. Lipids Res.,* 1967, **8**, 142. The mixture was mixed at 40°C for 24 hours. The tetrahydrofuran was then evaporated and the mixture was acidified to pH 1 with HCl, whereupon the crude product precipitated and was collected. After washing with water the product was crystallized from isopropanol.

### B: preparation of GSG

(a) 1 mole of sebacic acid was reacted with 2 moles of N-hydroxysuccinimide and 2 moles of dicyclohexyl carbondiimide in ethyl acetate. The resulting compound, 1,10 sebacoyl di (N-hydroxysuccinimide) ester, [Seb-(NHS)₂] was crystallized from isopropanol. Seb(NHS)₂ was found to have an m.p. of 159°C.
(b) 1 volume of 30mM Seb(NHS)₂ in tetrahydrofuran with 1 volume excess glycine (0.3 M) in aqueous 0.1 M sodium bicarbonate according to the procedure described by Lapidot *et al., supra.* After 24 hours of mixing at 40°C, the tetrahydrofuran was evaporated under reduced pressure and the product was precipitated by acidifying with 1M HCl to pH 1. The precipitate was collected and washed with water. Crystallization was from isopropanol.

### C: preparation of NSN

NSN was prepared by the same procedure of Example (B) except that instead of glycine in step (b), 1-asparagine was used.

The remaining compounds described in the following were prepared in a similar manner, *mutatis mutandis.*

### Example 2: Experimental results

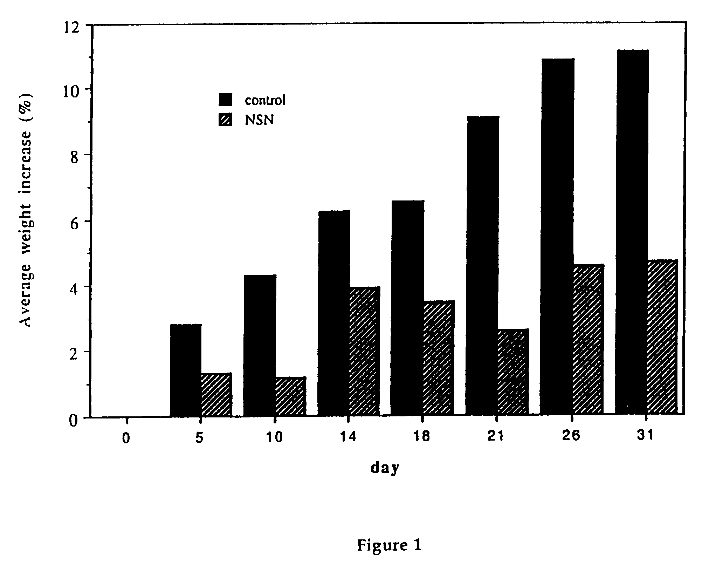

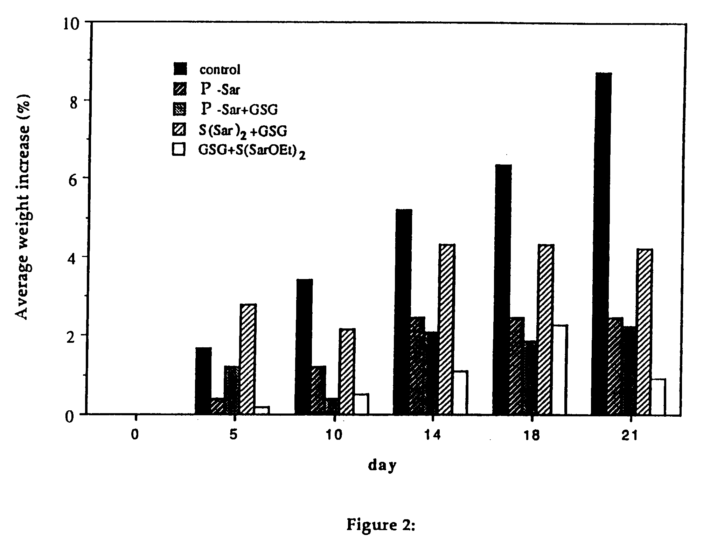

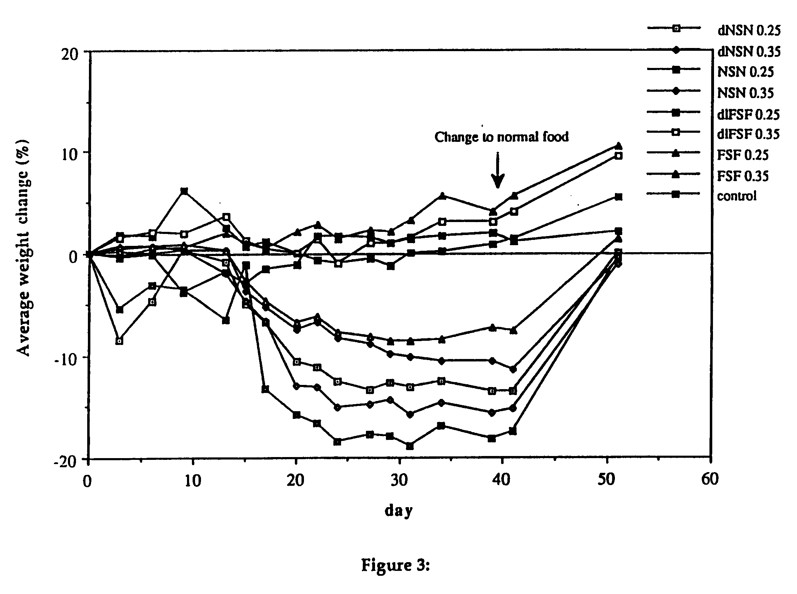

(a) Two groups of 5 three months old mice were fed with purina, *ad lib.* The diet of 1 group was supplemented with NSN (0.1% w/w in purina). The weight increase of the mice in each group was measured over 31 days and the results shown in Fig. 1 clearly demonstrate that the weight increase of the experimental group, was far less than that in the control group.
(b) 5 groups of 5 three months old mice each, were fed with a fattening diet consisting of purina and 6% corn oil, and out of these five groups, the diet of four was supplemented with anti-fattening drugs in accordance with the invention (0.1% w/w in the food). The following drugs were tested: P-Sar, GSG, S(Sar)₂ and S(SarOEt)₂.
   The results shown in Fig. 2 clearly demonstrate that the weight increase of the treated animals was far less than that of the animals of the control group.
(c) Adult mice were divided into 9 groups of five mice each, and were fed with normal purina *ad lib.* and 8 groups received one of the following supplements in their diet (0.25% or 0.35% w/w in the food): dNSN, NSN, dlFSF and FSF.
   One group did not receive any supplement and served as control.
   Food consumption was *ad lib.*
   The treatment was over a period of 40 days after which it was ceased and all groups of animals returned to a normal diet.
   The results shown in Fig. 3 clearly demonstrate that some of the supplements caused even a slight increase over control. Thus for example, while an increase was observed with 0.25% FSF, a considerable decrease in weight over the entire tested period was observed with 0.35%. FSF. Accordingly it is believed that upon increase of the concentration of these drugs they will all have an anti-fattening affect.
(d) 8-12 months old mice were administered with NSN either intra peritonelly (I.P.) or Per Os (P.O.), 10 mg per day for 4 days. The incorporation of ³H₂O to liver and adipose tissue was tested. For that purpose tritiated water was injected I.P. after overnight fast and 2 hours later the animals were sacrificed and the incorporation into lipids of the liver and the adipose tissue were determined by measuring radioactivity.

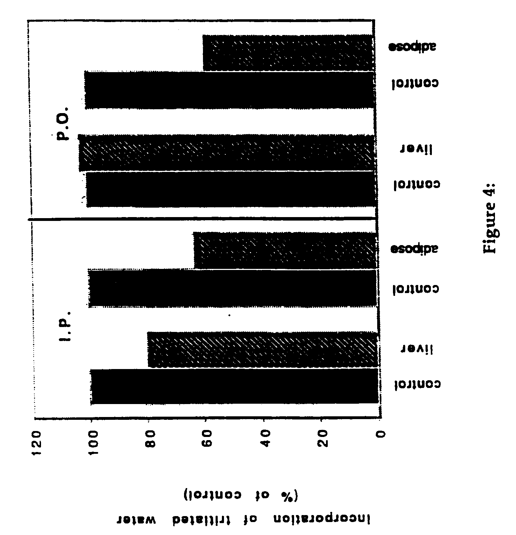

The results shown in Fig. 4, clearly demonstrate the decrease in treated water incorporation into the treated animals.

## Claims

1. Use of an active ingredient for the preparation of a pharmaceutical composition for the treatment of obesity, said active ingredient being a compound having the following general Formula I:
R₄-(CH₂)ₙ-CO-N(R₁)-CH(R₂)-CO(-R₃) (I)
where R₁ represents H or CH₃;
R₂ represents a side chain of a naturally occurring amino acid;
R₃ represents OH, OCH₂CH₃ and NH₂;
n is 6 - 18; and
R₄ represents CH₃ or a group having the general Formula II:
R₃-CO-CH(R₂)-N(R₁)-CO- (II)
where R₁, R₂ and R₃ have the above meanings.

2. Use according to Claim 1, wherein said compounds is a member of the group consisting of N-palmitoyl sarcosin, N,N'-sebacoyl bis-glycine, N',N-sebacoyl bis ℓ-asparagine, N,N'-sebacoyl bis d-asparagine N',N-sebacoyl bis sarcosin, N,N'-sebacoyl bis-sarcosin-ethylester and N,N'-sebacoyl bis-phenylalanine.

3. Use of an active ingredient for the preparation of a dietary supplement to be administered to obese individuals, said active ingredient being a compound having the general Formula I
R₄-(CH₂)ₙ-CO-N(R₁)-CH(R₂)-CO(-R₃) (I)
where R₁ represents H or CH₃;
R₂ represents a side chain of a naturally occurring amino acid;
R₃ represents OH, OCH₂CH₃ and NH₂;
n is 6 - 18; and
R₄ represents CH₃ or a group having the general Formula II:
R₃-CO-CH(R₂)-N(R₁)-CO- (II)
where R₁, R₂ and R₃ have the above meanings.

4. Use according to Claim 3, wherein in said compound R₄ represents a group of the general Formula II:
R₃-CO-CH(R₂)-N(R₁)-CO- (II)
where R₁, R₂ and R₃ have the above meanings.

5. A pharmaceutical composition comprising a pharmaceutically acceptable carrier, and, as an active ingredient, a compound having the following general Formula I
R₄-(CH₂)ₙ-CO-N(R₁)-CH(R₂)-CO(-R₃) (I)
where R₁ represents H or CH₃;
R₂ represents a side chain of a naturally occurring amino acid;
R₃ represents OH, OCH₂CH₃ and NH₂;
n is 6 - 18; and
R₄ represents a group having the general Formula II:
R₃-CO-CH(R₂)-N(R₁)-CO- (II)
where R₁, R₂ and R₃ have the above meanings.

## Patentansprüche

1. Verwendung eines Wirkstoffs zur Herstellung eines Arzneimittels zur Behandlung von Fettleibigkeit, wobei der Wirkstoff eine Verbindung der folgenden allgemeinen Formel I ist:
R₄-(CH₂)ₙ-CO-N(R₁)-CH(R₂)-CO(-R₃) (I)
in der R₁ ein Wasserstoffatom oder eine Methylgruppe darstellt;
R₂ eine Seitenkette einer natürlich vorkommenden Aminosäure darstellt;
R₃ eine OH-, OCH₂CH₃- oder NH₂-Gruppe darstellt;
n 6-18 ist; und
R₄ eine Methylgruppe oder einen Rest der allgemeinen Formel II darstellt:
R₃-CO-CH(R₂)-N(R₁)-CO- (II)
in der R₁, R₂ und R₃ die vorstehend angegebenen Bedeutungen haben.

2. Verwendung nach Anspruch wobei die Verbindungen ein Mitglied der Gruppe N-Palmitoylsarcosin, N,N'-Sebacoyl-bis-glycin, N',N-Sebacoyl-bis-ℓ-asparagin, N,N'-Sebacoyl-bis-d-asparagin, N',N-Sebacoyl-bis-sarcosin, N,N'-Sebacoyl-bis-sarcosinethylester und N,N'-Sebacoyl-bis-phenylalanin sind.

3. Verwendung eines Wirkstoffs zur Herstellung eines diätetischen Zusatzes, der fettleibigen Personen zu verabreichen ist, wobei der Wirkstoff eine Verbindung der allgemeinen Formel I ist
R₄-(CH₂)ₙ-CO-N(R₁)-CH(R₂)-CO(-R₃) (I)
in der R₁ ein Wasserstoffatom oder eine Methylgruppe darstellt;
R₂ eine Seitenkette einer natürlich vorkommenden Aminosäure darstellt;
R₃ eine OH-, OCH₂CH₃- oder NH₂-Gruppe darstellt;
n 6-18 ist; und
R₄ eine Methylgruppe oder einen Rest der allgemeinen Formel II darstellt:
R₃-CO-CH(R₂)-N(R₁)-CO- (II)
in der R₁, R₂ und R₃ die vorstehend angegebenen Bedeutungen haben.

4. Verwendung nach Anspruch 3, wobei in der Verbindung R₄ einen Rest der allgemeinen Formel II darstellt:
R₃-CO-CH(R₂)-N(R₁)-CO- (II)
in der R₁, R₂ und R₃ die vorstehend angegebenen Bedeutungen haben.

5. Arzneimittel, umfassend einen pharmazeutisch verträglichen Träger und als Wirkstoff eine Verbindung mit der folgenden allgemeinen Formel I
R₄-(CH₂)ₙ-CO-N(R₁)-CH(R₂)-CO(-R₃) (I)
in der R₁ ein Wasserstoffatom oder eine Methygruppe darstellt;
R₂ eine Seitenkette einer natürlich vorkommenden Aminosäure darstellt;
R₃ eine OH-, OCH₂CH₃- oder NH₂-Gruppe darstellt;
n 6-18 ist; und
R₄ einen Rest der allgemeinen Formel II darstellt:
R₃-CO-CH(R₂)-N(R₁)-CO- (II)
in der R₁, R₂ und R₃ die vorstehend angegebenen Bedeutungen haben.

## Revendications

1. Utilisation d'un principe actif pour la préparation d'une composition pharmaceutique pour le traitement de l'obésité, ledit principe actif étant un composé possédant la formule I générale suivante:
R₄-(CH₂)ₙ-CO-N(R₁)-CH(R₂)-CO(-R₃) (I)
dans laquelle
R₁ représente un atome d'hydrogène ou un groupe CH₃;
R₂ représente une chaîne latérale d'un amino-acide d'origine naturelle;
R₃ représente OH, OCH₂CH₃ et NH₂;
n vaut de 6 à 18; et
R₄ représente CH₃, ou un groupe possédant la formule générale II:
R₃-CO-CH(R₂)-N(R₁)-CO- (II)
dans laquelle
R₁, R₂ et R₃ possèdent les significations ci-dessus.

2. Utilisation selon la revendication 1, dans laquelle ledit composé fait partie du groupe formé par la N-palmitoyl-sarcosine, la N,N'-sébacoyl-bis-glycine, la N',N-sébacoyl-bis-ℓ-asparagine, la N,N'-sébacoyl-bis-d-asparagine, la N,'N-sébacoyl-bis-sarcosine, le N,N'-sébacoyl-bis-sarcosine-éthylester et la N,N'-sébacoyl-bis-phénylalanine.

3. Utilisation d'un principe actif pour la préparation d'un complément diététique à administrer à des individus obèses, ledit principe actif étant un composé, possédant la formule I générale:
R₄-(CH₂)ₙ-CO-N(R₁)-CH(R₂)-CO(-R₃) (I)
dans laquelle
R₁ représente un atome d'hydrogène ou un groupe CH₃;
R₂ représente une chaîne latérale d'un amino-acide d'origine naturelle;
R₃ représente OH, OCH₂CH₃ et NH₂;
n vaut de 6 à 18; et
R₄ représente CH₃, ou un groupe possédant la formule générale II:
R₃-CO-CH(R₂)-N(R₁)-CO- (II)
dans laquelle
R₁, R₂ et R₃ possèdent les significations ci-dessus.

4. Utilisation selon la revendication 3, dans laquelle ledit composé R₄ représente un groupe de formule générale II:
R₃-CO-CH(R₂)-N(R₁)-CO- (II)
dans laquelle
R₁, R₂ et R₃ possèdent les significations ci-dessus.

5. Composition pharmaceutique comprenant un véhicule acceptable sur le plan pharmaceutique, et, comme principe actif, un composé possédant la formule I générale suivante:
R₄-(CH₂)ₙ-CO-N(R₁)-CH(R₂)-CO(-R₃) (I)
dans laquelle
R₁ représente un atome d'hydrogène ou un groupe CH₃;
R₂ représente une chaîne latérale d'un amino-acide d'origine naturelle;
R₃ représente OH, OCH₂CH₃ et NH₂;
n vaut de 6 à 18; et
R₄ représente un groupe possédant la formule générale II:
R₃-CO-CH(R₂)-N(R₁)-CO- (II)
dans laquelle
R₁, R₂ et R₃ possèdent les significations ci-dessus.
